(19) Europäisches Patentamt
European Patent Office
Office européen des brevets

(11) Numéro de publication: **0 014 651**
**B1**

(12) # FASCICULE DE BREVET EUROPEEN

(45) Date de publication du fascicule du brevet: **04.08.82**

(51) Int. Cl.³: **C 07 D 499/22**, A 61 K 31/43

(21) Numéro de dépôt: **80400168.3**

(22) Date de dépôt: **04.02.80**

(54) **2-Méthoxyphénoxyacétate de talampicilline, procédé pour sa préparation et médicaments le renfermant.**

(30) Priorité: **05.02.79 IT 1989679**

(43) Date de publication de la demande: **20.08.80 Bulletin 80/17**

(45) Mention de la délivrance du brevet: **04.08.82 Bulletin 82/31**

(84) Etats contractants désignés: **DE FR IT**

(56) Documents cités: **FR-A-2 100 729**

(73) Titulaire: **SANOFI, société anonyme, 40, Avenue George V, F-75008 Paris (FR)**

(72) Inventeur: **Boveri, Sergio, Via C.Abba 5, I-20052 Monza (Milano) (IT)**
Inventeur: **Pedrazzoli, Andréa, Via Anelli 1, I-20100 Milano (IT)**

(74) Mandataire: **Combe, André et al, CABINET BEAU DE LOMENIE 55 rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

EP 0 014 651 B1

2-Méthoxyphénoxyacétate de talampicilline, procédé pour sa préparation et médicaments le renfermant

La présente invention concerne le 2-méthoxyphénoxyacétate de l'ester phtalidylique de l'acide 6-[D(–)-α-aminophénylacétamido] pénicillanique ainsi qu'un procédé pour sa préparation et des médicaments renfermant ledit produit en tant que principe actif.

L'ester phtalidylique de l'acide 6-[D(–)-α-aminophénylacétamido] pénicillanique de formule:

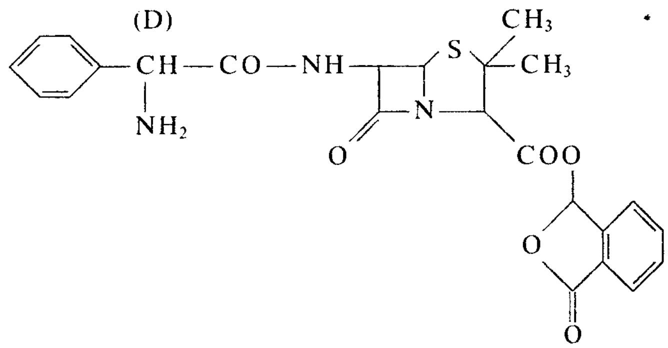

(I)

ci-après désigné »talampicilline«, est un ester utile de l'acide 6-[D(–)-aminophénylacétamido] pénicillanique, ci-après désigné »ampicilline«, qui est bien absorbé lorsqu'il est administré par voie orale chez l'homme et les animaux, en donnant ainsi de taux sanguins très élevés en ampicilline-mère. La talampicilline et ses sels, en particulier le chlorhydrate, sont indiqués dans le brevet anglais 1 364 672.

La demande de brevet italien 26 195 A/78 décrit l'ester phtalidylique de l'acide 6-[D(–)-α-hydroxyméthylaminophénylacétamido]pénicillanique de formule

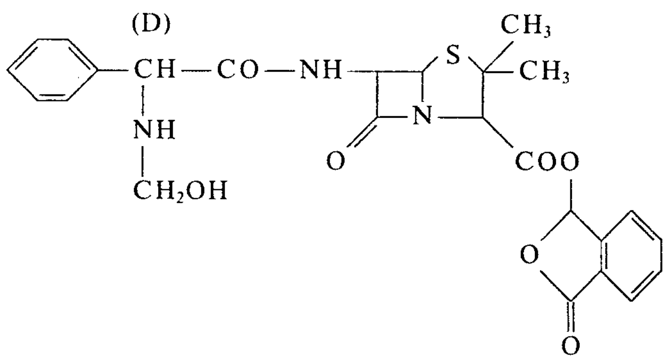

(II)

ci-après désigné »ester phtalidylique de la métampicilline« ainsi qu'un procédé pour sa préparation et pour sa conversion éventuelle en chlorhydrate de talampicilline.

Plus particulièrement, la demande de brevet italien cidessus se réfère à un procédé caractérisé en ce que l'on traite un sel alcalin ou d'ammonium de l'acide 6-[D(–)-α-méthylèneaminophénylacétamido]pénicillanique de formule

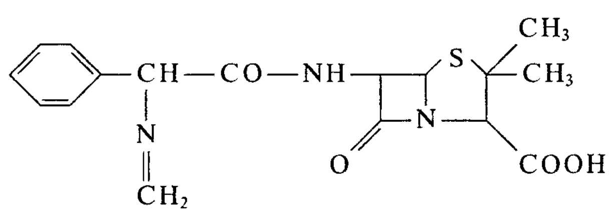

(III)

ou de l'acide 6-[D(-)-α-hydroxyméthylaminophénylacétamido] pénicillanique de formule

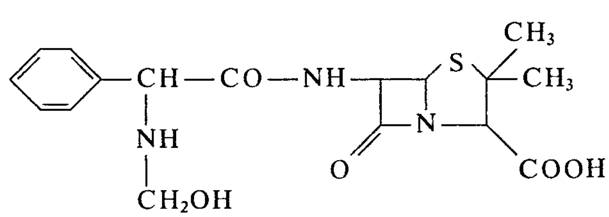

(IV)

les deux ci-après indifféremment désignés »métampicilline«, par un 3-halophtalide et l'on hydrolyse le produit ainsi obtenu dans un solvant aqueux-organique biphasique en présence d'acide chlorhydrique pour obtenir le chlorhydrate de talampicilline.

L'utilité de l'ester phtalidylique de métampicilline II et du procédé ci-dessus réside en ce qu'il permet d'obtenir un chlorhydrate de talampicilline de haute qualité avec un rendement pratiquement quantitatif à partier de sels de métampicilline à leur tour préparés avec un rendement pratiquement quantitatif à partier de sels d'ampicilline et formaldéhyde.

La demande de brevet italien 19 895 A/79 décrit un procédé pour la préparation du chlorhydrate de talampicilline selon le même procédé revendiqué par la demande de brevet italien 26 195 A/78, mais en partant de l'ampicilline et en agissant sur un sel alcalin ou d'ammonium de la métampicilline dans le même solution de préparation.

Le procédé des demandes de brevet ci-dessus, qui comprend, en tant qu'éléments clé, l'intermédiaire ester phtalidylique de métampicilline (formule II) ainsi que son hydrolyse avec de l'acide chlorhydrique dans un solvant aqueux-organique biphasique, permet l'obtention d'un chlorhydrate de talampicilline qui est isolé selon les techniques habituelles, par exemple par extraction avec un solvant organique et précipitation.

Selon ce qui est indiqué dans la demande de brevet italien 19 895 A/79, si l'on remplace l'acide chlorhydrique par un autre acide organique ou inorganique convenable, on isole le sel d'addition de talampicilline correspondant.

On a maintenant trouvé que le 2-méthoxyphénoxyacétate de talampicilline peut être préparé avec des rendements très élevés en hydrolysant l'ester phtalidylique de métampicilline II avec de l'acide chlorhydrique dans un solvant aqueux-organique biphasique et en traitant ensuite la phase aqueuse qui se sépare à la fin de la réaction par une solution aqueuse d'un sel alcalin de l'acide 2-méthoxyphénoxyacétique.

Ainsi, se sépare par précipitation le sel de talampicilline correspondant qui peut être isolé par simple filtration.

Le 2-méthoxyphénoxyacétate de l'ester phtalidylique de l'acide 6-[D(−)-α-aminophénylacétamido]pénicillanique, caractérisé par la formule

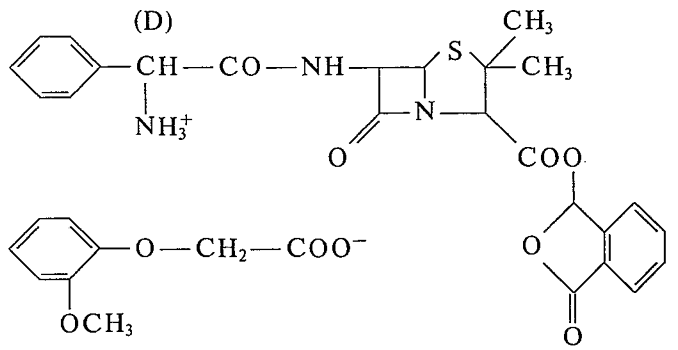

(V)

et ci-après désigné »gaïacolglycolate de talampicilline«, est un nouveau sel de talampicilline qui, par rapport aux autres sels de talampicilline a l'avantage d'être très stable et de permettre la préparation de comprimés ou de poudres à usage pharmaceutique sans précautions particulières.

Par rapport au napsylate de talampicilline décrit dans la demande de brevet européen publiée au n. 4 740, le gaïacolglycolate de talampicilline a l'avantage d'être plus facilement préparé car les sels de l'acide gaïacolglycolique, en particulier le sel de sodium, sont très solubles dans l'eau. Par contre, en particulier, le sel de sodium de l'acide 2-naphtalènesulfonique n'est soluble qu'à chaud (35 à 40°C) dans des volumes d'eau assez élevés avec formation, en général, de précipités qui doivent être éliminés par filtration.

En outre, le gaïacolglycolate de talampicilline est une poudre blanche qui peut être aisement isolée du mélange réactionnel par simple filtration et ne présente aucun problème de conservation. De ce point de vue, le composé de la présente invention est particulièrement avantageux.

Par rapport au chlorhydrate, le gaïacolglycolate de talampicilline a aussi l'avantage de ne pas avoir la saveur amère qui oblige à enrober les comprimés et de permettre la préparation de poudres pour de sirops extemporanés, tout en présentant une très bonne absorption dans l'organisme et donnant de taux sanguins très élevés d'ampicilline.

Selon le procédé de la présente invention, décrit dans les exemples ci-dessous, l'hydrolyse est effectuée avec de l'acide chlorhydrique parce que cet acide est plus facilement accessible et plus maniable, mais il est évident qu'on peut utiliser n'importe quel acide qui donne, à la fin de l'hydrolyse, un sel de talampicilline soluble dans l'eau, par exemple l'acide sulfurique ou l'acide méthanesulfonique. L'utilisation de tels acides, organiques ou inorganiques, entre dans le cadre de l'invention.

Le procédé de la présente invention peut être conduit sur un ester phtalidylique de la métampicilline

Il à l'état pur, ou bien en forme brute à la fin de sa préparation à partir d'un sel de métampicilline, comme décrit dans la demande de brevet italien 26 195 A/78, ou à partir de l'ampicilline anhydre ou trihydratée, ou aussi sodique, comme décrit dans la demande de brevet italien 19 895 A/79.

Un autre objet de la présente invention est une composition pharmaceutique contenant le gaïacolglycolate de talampicilline avec un excipient pharmaceutique.

Les compositions pharmaceutiques de la présente invention sont administrées de préference par voie orale sous forme de doses posologiques unitaires solides ou liquides telles que comprimés, gélules, poudres, granulés, sirops et similaires contenant les quantités appropriées de principe actif.

On prépare simplement les poudres en broyant le composé actif a une finesse convenable et en mélangeant avec un diluant broyé similairement. Le diluant peut être un matériau de glucide comestible tel que l'amidon. Il est avantageux qu'un agent édulcorant ou un sucre, ainsi qu'une huile parfumant, soit présent.

On prépare les granulés destinés à la reconstitution d'une préparation liquide orale en utilisant des diluants solubles dans l'eau. On mouille le composé actif et un diluant soluble dans l'eau tel que saccharose, glucose, etc. avec un liant tel que mucilage d'acacia, solution de gélatine, solution de méthylcellulose et on fait sur un tamis en forçant pour former des granulés que l'on fait sécher. Il est avantageux d'introduire dans la composition un agent de mise en suspension comme la gomme adragante.

On fait les gélules en préparant un mélange pulvérulent comme il est décrit ci-dessus et en le mettant dans des gaines de gélatine mises en forme. Comme adjuvant à l'opération de remplissage il est avantageux d'ajouter un lubrifiant tel que talc, stéarate de magnésium et stéarate de calcium au mélange pulvérulent avant l'opération de remplissage.

On fait les comprimés en préparant un mélange pulvérulent, en granulant ou en tronçonnant, en ajoutant un lubrifiant et en comprimant pour former des comprimés. On prépare le mélange pulvérulent en mélangeant le composé désiré, convenablement broyé, avec un diluant ou une base telle que amidon, saccharose, kaolin, phosphate bicalcique, etc. On peut granuler le mélange pulvérulent en mouillant avec le liant tel que sirop, pâte d'amidon ou mucilage d'acacia et en forçant à travers un tamis. Comme méthode autre que la granulation, on peut tronçonner le mélange pulvérulent, c'est-à-dire, le faire passer à travers la machine à comprimés et casser les comprimés résultant imparfaitement formés en fragments (tronçons). On peut lubrifier les tronçons pour les empêcher de coller aux comprimés en formant des cubes, en ajoutant un sel stéarate, du talc ou une huile minérale. On comprime ensuite le mélange lubrifiant pour constituer des comprimés.

Le comprimé peut être avantageusement muni d'un revêtement protecteur constitué d'un enduit de scellement de gomme-laque, un revêtement de sucre et de méthylcellulose, et un enduit vernis de cire de carnauba.

On prépare les fluides pour administration orale sous des formes posologiques unitaires tels que sirops où chaque cuillère à café de composition contient une quantité prédéterminée du composé actif à administrer.

On prépare un sirop en mettant le composé actif en suspension dans une solution aqueuse de saccharose convenablement parfumée.

Grâce aux propriétés organoleptiques du gaïacolglycolate de talampicilline, les compositions préférentielles sont des granulés destinés à la reconstitution de préparations liquides orales, telles que les sirops extemporanés, mais des compositions sous forme de comprimés sont egalement avantageuses.

Les compositions de la présente invention contiennent de 20 a 1000 mg de gaïacolglycolate de talampicilline par unité de dosage. Des unités de dosage préférentielles contiennent 160, 320 ou 480 mg de gaïacolglycolate de talampicilline correspondant à 125, 250 ou, respectivement, 375 mg de chlorhydrate de talampicilline. Lesdites compositions sont utiles pour le traitement des infections provoquées par des micro-organismes pathogènes sensibles à l'ampicilline.

Les exemples suivants illustrent l'invention sans toutefois la limiter.

## Exemple 1

A une solution de 20,4 g d'ester phtalidylique de la métampicilline dans 100 ml d'acétate d'éthyle on ajoute une solution de 40 ml d'acide chlorhydrique N dans 120 ml d'eau. On maintient le mélange sous bonne agitation pendant 45 minutes à la température ambiante, puis on sépare la phase organique et l'on lave deux fois la phase aqueuse avec de l'éther diéthylique. On laisse la solution aqueuse pendant 20 minutes à 20° C sous pression réduite, puis on la filtre sur de l'amiante traité à l'acide chlorhydrique. A la solution limpide ainsi obtenue on ajoute une solution de 8 g d'acide gaïacolglycolique dans 100 ml d'eau contenant 1,76 g d'hydroxyde de sodium en la faisant dégoutter rapidement sous agitation. On filtre le précipité qui s'est formé, on le lave à l'eau et on le sèche sur $P_2O_5$ à la température ambiante et sous pression réduite. On obtient ainsi le gaïacolglycolate de talampicilline; p. f. 105 à 110° C (dec.).

Rendement: 82,5% de la théorie.

Exemple 2

A une suspension de 262 g d'ampicilline anhydre dans 1850 ml de diméthylformamide on ajoute, à la température de 5°C et sous agitation, 62 ml d'une solution aqueuse de formaldéhyde à 40%, ensuite, 54 g de carbonate de potassium anhydre. On agite le mélange pendant 90 minutes à une température entre 0 et 5°C et puis on la traite par 160 g de 3-bromophtalide. On maintient le mélange réactionnel sous agitation à 10—13°C pendant encore 4 heures, puis on le verse dans 5 litres d'eau glacée. On filtre le précipité qui s'est formé, on le lave avec 1000 ml d'eau froide et on le dissout dans 1700 ml d'acétate d'éthyle. On élimine l'eau résiduelle, on traite la solution organique par 760 ml d'acide chlorhydrique N et 2500 ml d'eau et l'on agite le mélange pendant 45 minutes à la température ambiante. On sépare la couche aqueuse, on la lave encore une fois avec 200 ml d'éther diéthylique, on la maintient sous pression réduite à 20°C pendant 20 minutes, puis on la filtre sur de l'amiante prétraité par de l'acide chlorhydrique. On agite la solution limpide ainsi obtenue, dont le volume est 3500 ml environ, et on y fait dégoutter, pendant environ 10 minutes, une solution de 145 g d'acide gaïacolglycolique dans 1800 ml d'eau contenant 32 g d'hydroxyde de sodium. On laissesous agitation pendant encore 5 minutes le précipité qui s'est formé, puis on le filtre, on le lave avec 500 ml d'eau et on le sèche sur $P_2O_5$ à la température de 25 à 30°C sous pression réduite. On obtient ainsi le gaïacolglycolate de talampicilline identique au produit décrit à l'exemple 1.

Exemple 3

On prépare une composition pour comprimés ayant la formule suivante:

| | |
|---|---|
| gaïacolglycolate de talampicilline | 480 mg |
| glycine | 130 mg |
| cellulose microgranulaire | 70 mg |
| silice précipitée | 19 mg |
| carboxyméthylamidon | 35 mg |
| stéarate de magnésium | 13 mg |
| talc | 13 mg |

On mélange les quantites calculées des composants pendant 30 minutes, puis on granule à sec et on fait passer le mélange à travers un tamis à mailles de 1,6 mm. On comprime ensuite en utilisant un poinçon ayant la forme de petit bâton. On obtient ainsi des comprimés pésant chacun 760 mg et renfermant chacun 480 mg de principe actif.

Exemple 4

On prépare des comprimés selon l'exemple 3. Les comprimés ainsi obtenus sont enrobés à l'aide d'une suspension de pthalate de dibutyle, de polyméthacrylate de butyle et diméthylaminoéthyle, de polyéthylèneglycol 1500, de silice précipitée, de bioxyde de titanium et de talc dans un mélange acétone/isopropanol 1 : 1 ayant une concentration en résidu sec de 10% environ. On obtient ainsi des comprimés enrobés pésant chacun 780 mg et renfermant chacun 480 mg de gaïacolglycolate de talampicilline.

Exemple 5

On prépare un granulé destiné à la reconstitution d'une préparation liquide orale ayant la composition suivante:

| | |
|---|---|
| gaïacolglycolate de talampicilline | 3,6 g |
| saccharose | 50,0 g |
| carboxyméthylcellulose sodium | 0,8 g |
| acide citrique | 0,1 g |
| citrate trisodique | 0,9 g |
| benzoate de sodium | 0,25 g |
| saccharine sodique | 0,15 g |
| aromatisant | 0,5 g |

Le volume du granulé ainsi obtenu est porté à 100 ml avec de l'eau pour sirops. Une dose unitaire de 5 ml du sirop extemporané ainsi obtenu contient 160 mg de gaïacolglycolate de talampicilline.

Pour préparer le granulé, on pulverise les quantités calculées de tous les composants, sauf le

sacharose, puis on mélange la poudre ainsi obtenue avec le saccharose jusqu'à obtenir un granulé homogène.

Exemple 6

On prépare selon l'Exemple 5 un granulé destiné à la reconstitution d'une préparation liquide orale ayant la composition suivante:

| | |
|---|---|
| gaïacolglycolate de talampicilline | 7,2 g |
| saccharose | 46,4 g |
| carboxyméthylcellulose sodium | 0,9 g |
| acide citrique | 0,1 g |
| citrate trisodique | 0,9 g |
| benzoate de sodium | 0,25 g |
| saccharine sodique | 0,15 g |
| aromatisant | 0,5 g |

Une dose unitaire de 5 ml du sirop extemporané ainsi obtenu contient 320 mg gaïacolglycolate de talampicilline.

Exemple 7

On prépare selon l'Exemple 5 un granulé destiné à la reconstitution d'une préparation liquide orale ayant la composition suivante:

| | |
|---|---|
| gaïacolglycolate de talampicilline | 9,6 g |
| saccharose | 44,0 g |
| carboxyméthylcellulose sodium | 1,0 g |
| acide citrique | 0,1 g |
| citrate trisodique | 0,9 g |
| benzoate de sodium | 0,25 g |
| saccharine sodique | 0,15 g |
| aromatisant | 0,5 g |

Une dose unitaire de 5 ml du sirop extemporané ainsi obtenu contient 480 mg de gaïacolglycolate de talampicilline.

**Revendications**

1. Le 2-méthoxyphénoxyacétate de l'ester phtalidylique de l'acide 6-[D(−)-α-aminophénylacétamido]pénicillanique de formule

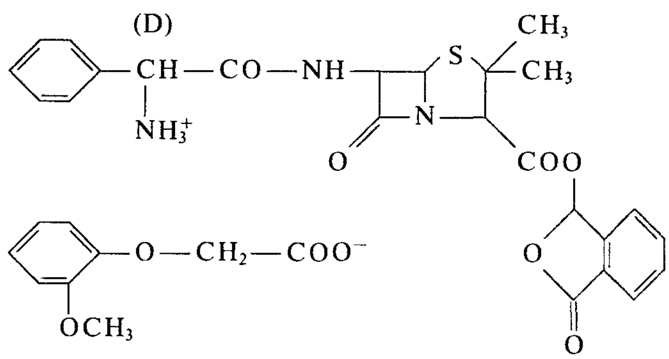

2. Procédé pour la préparation du 2-méthoxyphénoxyacétate de l'ester phtalidylique de l'acide 6-[D(−)-α-aminophénylacétamido]pénicillanique, caractérisé en ce que l'on traite l'ester phtalidylique de la métampicilline avec un acide approprié dans un solvant aqueux-organique biphasique, on traite la phase aqueuse résultante avec une solution aqueuse d'un sel alcalin de l'acide 2-méthoxyphénoxyacétique et on isole par filtration le produit ainsi obtenu.

3. Procédé selon la revendication 2 caractérisé en ce que comme sel alcalin de l'acide 2-méthoxyphénoxyacétique on utilise le sel de sodium ou de potassium.

4. Procédé selon les revendications 2 et 3 caractérisé en ce que comme solvant aqueux-organique biphasique on utilise un mélange eau/acétate d'éthyle.
5. Composition pharmaceutique contenant le 2-méthoxyphénoxyacétate de l'ester phtalidylique de l'acide 6-[D(—)-α-aminophénylacétamido]pénicillanique avec un excipient pharmaceutique.
6. Composition pharmaceutique selon la revendication 5, sous forme de granules destinés à la reconstitution de préparation liquides orales.
7. Composition selon les revendications 5 et 6 contenant 160 mg de 2-méthoxyphénoxyacétate de l'ester phtalidylique de l'acide 6-[D(—)-α-aminophénylacétamido]pénicillanique par unité de dosage.
8. Composition selon les revendications 5 et 6 contenant 320 mg de 2-méthoxyphénoxyacétate de l'ester phtalidylique de l'acide 6-[D(—)-α-aminophénylacétamido]pénicillanique par unité de dosage.
9. Composition selon les revendication 5 et 6 contenant 480 mg de 2-méthoxyphénoxyacétate de l'ester phtalidylique de l'acide 6-[D(—)-α-aminophénylacétamido]pénicillanique par unité de dosage.

## Patentansprüche

1. 2-Methoxyphenoxyacetat von 6-[D(—)-α-Aminophenylacetamido]-penicillansäure-phtalidylester der Formel

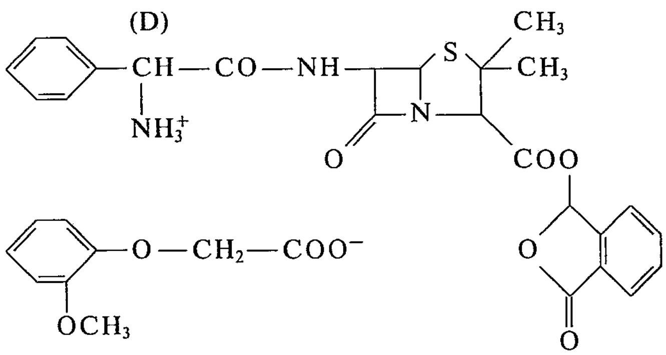

2. Verfahren zur Herstellung von 2-Methoxyphenoxyacetat von 6-[D(—)-α-Aminophenylacetamido]-penicillansäure-phtalidylester, dadurch gekennzeichnet, daß man das Phtalidylester von Metampicilline in einer zweiphasigen organisch-wässerigen Lösung mit einer geeigneten Säure behandelt, man die erhaltene wässerige Phase mit einer wässerigen Lösung eines Alkalisalzes von 2-Methoxyphenoxyacetansäure behandelt und den so erhaltenen Stoff durch Filtrierung isoliert.
3. Verfahren nach Anspruch 2, dadurch gekennzeichnet, daß als Alkalisalz der 2-Methoxyphenoxyacetansäure Natrium- oder Kaliumsalz verwendet wird.
4. Verfahren nach den Ansprüchen 2 und 3, dadurch gekennzeichnet, daß als zweiphasige organisch-wässerige Lösung ein Äthyl-Wasser/Acetatgemisch verwendet wird.
5. Pharmazeutische Verbindung aus 2-Methoxyphenoxyacetat von 6-[D(—)-α-Aminophenylacetamido]-penicillansäure-phtalidylester mit einem pharmazeutischen Bindemittel.
6. Pharmazeutische Verbindung nach Anspruch 5, in Form von Granula zur Zubereitung von flüssigen Präparaten zum Trinken.
7. Verbindung nach den Ansprüchen 5 und 6 aus 160 mg 2-Methoxyphenoxyacetat von 6-[D(—)-α-Aminophenylacetamido]-penicillansäure-phtalidylester pro Dosierungseinheit.
8. Verbindung nach den Ansprüchen 5 und 6 aus 320 mg 2-Methoxyphenoxyacetat von 6-[D(—)-α-Aminophenylacetamido]-penicillansäure-phtalidylester pro Dosierungseinheit.
9. Verbindung nach den Ansprüchen 5 und 6 aus 480 mg 2-Methoxyphenoxyacetat von 6-[D(—)-α-Aminophenylacetamido]-penicillansäure-phtalidylester pro Dosierungseinheit.

**Claims**

1. The 2-methoxyphenoxyacetic acid salt of the phthalidyl ester of the 6-[D(−)-α-aminophenylacetamido]penicillanic acid of the formula

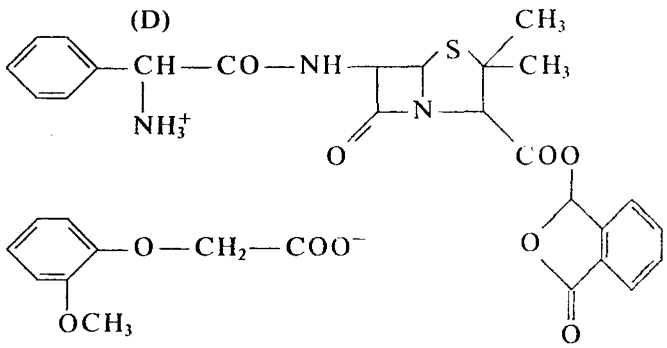

2. Process for the preparation of the 2-methoxyphenoxyacetate of the phthalidyl ester of the 6-[D(−)-α-aminophenylacetamido]penicillanic acid, characterized in that the metampicillin phthalidyl ester is treated with an appropriate acid in a biphasic aqueous-organic solvent, treating the resulting aqueous phase with an aqueous solution of an alkali metal salt of the 2-methoxy-phenoxyacetic acid and isolating the product thus obtained by filtration.

3. Process according to claim 2, characterized in that sodium or potassium salt is used as alkaline salt of the 2-methoxyphenoxyacetate.

4. Process according to claims 2 and 3, characterized in that a mixture water/ethyl acetate is used as a biphasic aqueous-organic solvent.

5. Pharmaceutical composition containing the 2-methoxyphenoxyacetate of the phthalidyl ester of 6-[D(−)-α-aminophenylacetamido]penicillanic acid with a pharmaceutical excipient.

6. Pharmaceutical composition according to claim 5 in the form of granules destined for the reconstitution of liquid oral preparations.

7. Pharmaceutical composition according to claims 5 and 6 containing 160 mg 2-methoxyphenoxyacetate of the phthalidyl ester of 6-[D(−)-α-aminophenylacetamido]penicillanic acid per dosage unit.

8. Pharmaceutical composition according to claims 5 and 6 containing 320 mg of 2-methoxyphenoxyacetate of the phthalidyl ester of 6-[D(−)-α-aminophenylacetamido]penicillanic acid per dosage unit.

9. Pharmaceutical composition according to claims 5 and 6 containing 480 mg of 2-methoxyphenoxyacetate of the phthalidyl ester of 6-[D(−)-α-aminophenylacetamide]penicillanic acid per dosage unit.